# EUROPEAN PATENT APPLICATION

(11) **EP 1 811 025 A1**
(43) Date of publication of application: **25.07.2007**
(21) Application number: 05793045.5
(22) Date of filing: 06.10.2005
(51) Int. Cl.: C12N 15/09, A01K 67/027, A61K 31/7088, C12N 5/10, A61K 39/395, A61K 45/00, A61K 48/00, A61P 25/28, A61P 43/00, C07K 7/06, C07K 7/08, C07K 14/47, C07K 16/18, A61K 38/00, C12Q 1/02, G01N 33/15, G01N 33/50, G01N 33/68

(54) **MUTATED AMYLOID PROTEIN**

(30) Priority: 06.10.2004 JP 2004294292
(71) Applicant: Mori, Hiroshi, Osaka-shi, Osaka 537-0025 (JP)
(72) Inventor: TOMIYAMA, Takami, Sakai-shi, Osaka 590-0137 (JP); SHIMADA, Hiroyuki, Osaka-shi, Osaka 535-0031 (JP); MORI, Hiroshi, Room 301, Haitsu-kosumo-21, Osaka-shi, Osaka 537-0025 (JP)
(74) Representative: Gardner, Rebecca Katherine
(86) International application number: PCT/JP2005/018896
(87) International publication number: WO 2006/038729

(57) **Abstract**

It is intended to provide a novel amyloid protein (human variant amyloid protein) useful as an antigen molecule or the like for improving a vaccine therapy. This human variant amyloid protein is a protein with deletion of Glu at 22nd, Ala at 21st, or Asp at 23rd in a normal (wildtype) amyloid protein (Aβ1-40. Aβ1-42, or Aβ1-43) consisting of 40, 42, or 43 amino acids.

## Description

### Technical Field

This invention relates to a variant amyloid protein existing in familial Alzheimer's disease patients as well as to utilization and development of such variant amyloid protein.

### Background Art

An amyloid protein is a type of tissue lesion that is observed in cerebral tissue of not only patients with Alzheimer's disease and Down's syndrome but also subjects with normal aged. The amyloid protein consists of 40 to 42 or 43 amino acids rich in hydrophobic amino acid and can be produced by hydrolytic cleavage of an amyloid precursor protein (hereinafter referred to as APP) which is a precursor thereof. APP consists of 695, 751, or 770 amino acids, which is a type-1 membrane protein with the single membrane domain, and has its amino terminal outside the cell. The difference in number of amino acids depends on the presence or absence of a protease inhibitor active site of a so-called Kunitz-type in the extracellular region.

A main component of neuron is APP consisting of 695 amino acids (hereinafter referred to as APP695). In turn, APP consisting of 770 amino acids (hereinafter referred to as APP770 of which cDNA base sequence and amino acid sequence are shown as SEQ ID NO 3) includes an amino acid sequence (75 amino acids from Glu to Lys at position 363 of SEQ ID NO 3) which is not included in molecular specie of APP695. APP770 is a molecular specie having the amino acid sequence identical to that of APP695 other than the above-identified insertion amino acid sequence. An isoform called APP751 is known in addition to APP695 and APP770, and this APP consists of 751 amino acids and lacks in 19 amino acids (Met at position 345 to Lys at position 363) of APP770. The amino acid sequence (56 amino acids from Glu at position 289 to Ala at position 344 in the amino acid sequence of APP770) inserted into both of APP751 and APP770 has Kunitz-type protease inhibitor activity and is considered to be expressed in cells other than the neuron.

APP695 is an isoform expressing mainly in the neuron and is a type-I membrane protein capable of passing through the cell membrane once and having a transmembrane domain (24 amino acids from glycine at position 625 to leucine at position 648). The amyloid protein consists of a shorter protein molecular specie having 40 amino acids of APP 695 (SEQ ID NO 2) from asparaginic acid at position 597 to valin at position 636 existing outside the cell and a longer protein molecular specie having 42 or 43 amino acids terminating at alanine at position 638 or threonine at position 639.

Bioactivity of the amyloid protein has been experimentally proved and its neuron toxicity is considered to be a key factor for onset of the Alzheimer's disease (Non-Patent Documents 1 and 2). In order to produce the amyloid protein from APP, tow-stage important reactions are necessary. In the first stage, the amino terminal of the amyloid protein is separated by APP cleavage by β secretase. In the second stage, the carboxyl terminal of the amyloid protein is cleaved by cleavage by γ secretase, thereby causing the dissociation of amyloid protein and the cytoplasmic fragment of APP. According to conventional findings, it has been considered that the second stage cleavage occurs at the gamma (γ) position which is the carboxyl terminal of the amyloid protein (between Val at position 711 and Ile at position 712, between Ala at position 713 and Thr at position 714, or between Thr at position 714 and Val at position 715 of APP770 of SEQ ID NO 3); however, it has recently been reported that the second stage cleavage occurs at the epsilon (ε) position (between Thr at position 719 and Leu at position 720 or between Leu at position 720 and Val at position 721 of APP770 of SEQ ID NO 3) which is near the cytoplasm and downstream from the amino acid residue 5 by 10 amino acids (in the carboxyl terminal direction).

The intracerebral neuron lesion of the Alzheimer's disease occurs in advance of clinical abnormal symptoms such as disorientation, memory decline, amnesia, decline in intellect, and behavior disorder. The neuron lesion includes amyloid protein deposition, neural fibril alteration, and cell dropout, and the amyloid protein deposition is the initial pathological reaction.

It is considered that the amyloid hypothesis, in which the amyloid protein production and deposition are hypothecated to be the causes of the Alzheimer's disease, is important to understand the progress of the Alzheimer's disease, and one of the grounds for the importance is found in familial Alzheimer's disease study.

Concept that the γ secretase is a gene product of presenilin 1 (Non-Patent Document 3) and presenilin 2 (Non-Patent Documents 4 and 5) present in chromosome 14, which have been discovered to be responsible genes for early onset familial Alzheimer's disease, has widely been accepted; however, this concept has not been completed.

Since APP is also a responsible gene for the early onset familial Alzheimer's disease (Non-Patent Document 6), it is demonstrated that plural factors are involved in etiology of the Alzheimer's disease which is a type of dementia.

As described in the foregoing, the amyloid protein consists of two components. One of them is the shorter amyloid protein component (hereinafter referred to as Aβ1-40) starting from asparaginic acid to terminate at valin at position 40, and the other is the longer amyloid protein component which consists of 42 or 43 amino acids starting from asparaginic acid (same as Aβ1-40 consisting of 40 amino acids) and is longer than Aβ1-40 by amino acids of two or three residues to terminate at Ala at position 42 or Thr at position 43 (hereinafter referred to as Aβ1-42 or Aβ1-43, or collectively Aβ1-42/43). In the longer components, Aβ1-42 is higher in hydrophobic property and more insoluble. A fiber-like amyloid fibril having a diameter of 5 to 6 nm is formed of the longer component and the shorter component wherein the longer component serves as a nucleus and the shorter component is attached to the nucleus.

It has been demonstrated that production of Aβ1-42/43 which is the longer amyloid protein is increased when a gene variant is present in presenilin 1 or presenilin 2. It is considered that the longer amyloid protein component which is considered to be a determinant of a threshold value for amyloid protein polymerization is formed in large quantity due to this variant effect, thereby accelerating the etiologic reaction.

Many mutation variants have been identified in APP, and typical variants are generally classified into Sweden variant (Met670Asn, Lys671Leu in accordance with the amino acid numbering of APP770; the same applies to the following variants) preceding the amino terminal of the amyloid protein, Dutch variant (Glu693Gln), London variant (Val717Ile), and Australia variant (Leu723Pro). Production of the two amyloid protein components are increased in Sweden variant, and only the production of Aβ1-42/43 is increased in London variant and Australia variant. Significance of Dutch variant is in the middle of discussion, and the conclusion has not been made yet.

E4 is known as a risk factor allele of apolipoprotein E. It has been proved statistically that the onset of Alzheimer's disease is set ahead by 8 to 10 years in the case where only one of chromosome alleles is E4 or by 16 to 20 years in the case where both of chromosome alleles are E4 (Non-Patent Document 7).

It is considered that the activity inhibition of β secretase and γ secretase suppresses the amyloid protein production and can be used as a therapeutic drug for stopping or delaying the progress of Alzheimer's disease. An α secretase reaction occurs in addition to the β secretase reaction in the first stage of hydrolysis of APP, and the γ secretase reaction occurs in the second stages of the α secretase reaction and the β secretase reaction; therefore, a wider spectral effect of the γ secretase reaction is expected.

Though an ultimate conclusion for identification of the γ secretase has not been obtained yet, it has been proved that presenilin 1 has the important role. According to experiments of animals and experiments in cultured cells from which the action of presenilin 1 is inactivated, it is reported that abnormalities in cerebral nerve generation, spine formation, or lymph cell generation are caused. That is, presenilin 1 has various effects other than the amyloid protein.

It is necessary to consider that the nonspecific activity suppression of the γ secretase can induce a severe adverse reaction such as induction of malignant transformation (Non-Patent Document 8). In turn, it has been suggested that non-steroidal anti-inflammatory drugs epidemiologically have an anti-dementia effect, and it has been proved that the non-steroidal anti-inflammatory drugs selectively inhibit the productions of Aβ1-42/43 (Non-Patent Documents 9 and 10) and have a function of Rho kinase (Non-Patent Document 11), thereby becoming an important anti-dementia drug candidate. However, concentrations in usage and the like still require examination.

Though the vaccine therapy (Non-Patent Document 12) using a synthetic peptide Aβ1-42 as an antigen has been studied as a promising treatment method, an adverse reaction of causing a severe encephalitis has been pointed out as a side-effect (Non-Patent Document 13). In relation to the vaccine therapy, the indirect vaccine therapy or the indirect immunotherapy using an anti-amyloid antibody (Non-Patent Document 14) has attracted attention, but they require further examination from clinical point of view. It has been pointed out that the adverse effect of the vaccine therapy can be reduced by changing the antigen position of the amyloid protein used as the antigen to the amino terminal of the Aβ4-10, inducing the IgG2b isotype antigen, intestine immunization through oral administration, or the like, but such methods have not been confirmed as ameliorative measures.

### List of Non-Patent Documents

1. Yankner,B.A. et al., Neurotoxicity of a fragment of the amyloid precursor associated with Alzheimer's disease. (1989) Science. 245(4916): 417-20.
2. Yankner, B.A., Neurotrophic and neurotoxic effects of amyloid beta protein: reversal by tachykinin neuropeptides. (1990) Science. 1990 250(4978): 279-82.
3. Sherrington, R. et al., Cloning of a gene bearing missense mutations in early-onset familial Alzheimer's disease. (1995) Nature, 375(6534), 754-760.
4. Levy-Lahad, E. et al., Candidate gene for the chromosome 1 familial Alzheimer's disease locus. (1995) Science, 269(5226), 973-977.
5. Rogaev, E.I. et-al. Familial Alzheimer's disease in kindreds with missense mutations in a gene on chromosome 1 related to the Alzheimer's disease type 3 gene. (1995) Nature 376(6543), 775-778.
6. Goate, A. et al., Segregation of a missense mutation in the amyloid precursor protein gene with familial Alzheimer's disease. (1991) Nature, 349(6311), 704-6.
7. Corder, E.H. et al., Gene dose of apolipoprotein E type 4 allele and the risk of Alzheimer's disease in late onset families. (1993) Science. 261(5123): 921-3.
8. Hardy, J. and Israel, A. Alzheimer's disease. In search of gamma-secretase. (1999) Nature. 398(6727), 466-7.
9. Lim, G.P. et al., Ibuprofen suppresses plaque pathology and inflammation in a mouse model for Alzheimer's disease. (2000) J Neurosci 20:5709-14.
10. Weggen, S. et al., A subset of NSAIDs lower amiloidgenic Abeta42 indipendently of cyclooxygenase activity. (2001) Nature 414:212-6.
11. Zhou, Y. et al., Nonsteroidal anti-inflammatory drugs can lower amyloidogenic A β 42 by inhibiting Rho. (2003) Science 302:1215-7.
12. Schenk, D., et al. Immunization with amyloid b-attenuates Alzheimer-disease-like pathology in the PDAPP mouse. (1999) Nature. 400, 173-6.
13. Nicoll, J.A.R. et al., Neuropathology of human Alzheimer disease after immunization with amyloid- peptide: a case report. (2003) Nature Med. 9: 448-452.
14. DeMattos et al. (2002) Science295: 2264-7.; Pfeifer, M. et al. Cerebral hemorrhage after passive anti-Aβ immunotherapy. (2002) Science 298: 1379-.

### Disclosure of the Invention

This invention has been accomplished in view of the above-described problems of the conventional technologies, and an object thereof is to provide a novel amyloid protein (human variant amyloid protein) useful as an antigen molecule and the like for improving the vaccine therapy.

Another object of this invention is to provide a diagnostic measure for amyloid diseases by using the above variant amyloid protein and gene materials encoding the variant amyloid protein and a material for the diagnostic measure.

Still another object of this invention is to provide a method for screening a therapeutic agent component effective for the amyloid diseases based on production mechanism of the variant amyloid protein.

Yet another object of this invention is to provide an anti-amyloid disease drug using the variant amyloid protein or the gene material thereof.

This invention provides the following inventions for solving the above-described problems.

A first invention is a variant amyloid protein, which consists of the following amino acid sequence taken from SEQ ID NO: 2:
(1) the 39 amino acids from position 597 to position 636 with the deletion of glutamic acid at position 618;
(2) the 41 amino acids from position 597 to position 638 with the deletion of glutamic acid at position 618;
(3) the 42 amino acids from position 597 to position 639 with the deletion of glutamic acid at position 618;
(4) the 39 amino acids from position 597 to position 636 with the deletion of alanine at position 617;
(5) the 41 amino acids from position 597 to position 638 with the deletion of alanine at position 617;
(6) the 42 amino acids from position 597 to position 639 with the deletion of alanine at position 617;
(7) the 39 amino acids from position 597 to position 636 with the deletion of aspartic acid at position 619;
(8) the 41 amino acids from position 597 to position 638 with the deletion of aspartic acid at position 619;
(9) the 42 amino acids from position 597 to position 639 with the deletion of aspartic acid at position 619.

More specifically, the variant amyloid protein of the first invention is a protein with deletion of Glu at 22nd, Ala at 21st, or Asp at 23rd in the normal (wildtype) amyloid protein consisting of 40, 42, or 43 amino acids (Aβ1-40, Aβ1-42, Aβ1-43). In the following description, the variant amyloid proteins consisting of the amino acid sequences (1) to (9) are sometimes referred to as follows: (1) Aβ1-40 (Δ22E), (2) Aβ1-42 (Δ22E), (3) Aβ1-43 (Δ22E), (4) Aβ1-40 (Δ21A), (5) Aβ1-42 (Δ21A), (6) Aβ1-43 (Δ21A), (7) Aβ1-40 (Δ23D), (8) Aβ1-42 (Δ23D), (9) Aβ1-43 (Δ23D).

Note that the variant amyloid proteins (Aβ1-40 (Δ22E), Aβ1-42 (Δ22E), Aβ1-43 (Δ22E)) are deletion variant amyloid proteins that the inventors isolated and identified from familial Alzheimer's disease patients. The variant amyloid proteins of the amino acid sequences (4) to (9) are presumed with high probability to have a function similar to the variant amyloid proteins of the amino acid sequences (1) to (3). More specifically, by an analysis of structure of a normal type or wildtype amyloid protein with the use of an existing protein structure prediction program (for example, a protein second structure prediction program included in Chou-Fasman 2^{nd} Structure Prediction Program: GENETXY version 8.1.8, product of GENETXY Co., Tokyo), it is confirmed that two α helixes shed by an irregular coil structure at about the 7^{th} amino acid exist in the 1^{st} to 25^{th} amino acids and that a β structure exists in a region from the 30^{th} amino acid to the carboxyl terminal. That is, the structure of the normal or wildtype amyloid protein is "α helix - a helix - β structure". In turn, by analyses of Dutch variant (Glu693Gln) which is the known variant amyloid protein and the variant amyloid proteins (Aβ1-40 (Δ22E), Aβ1-42 (Δ22E), Aβ1-43 (Δ22E)) of this invention consisting of the amino acid sequences (1) to (3) in the same manner as described above, it is confirmed that a structure of these variant amyloid proteins is "α helix - β structure - β structure". Therefore, the variant amyloid proteins of this invention are characterized by having the structure of "α helix - β structure - β structure", and the variant amyloid protein (Δ21A or Δ23D) from which the amino acid residue (Ala or Asp) preceding or following Glu at position 22 is deleted has the structure of "α helix - β structure - β structure". This fact is apparently different from the fact that a structure of a variant amyloid protein from which an amino acid secondarily preceding or following Glu at position 22 (Phe and Val) has the structure "α helix - a helix - β structure) same as that of the normal (wildtype) amyloid protein.

Note that the variant amyloid protein of the first invention includes proteins from which one or more amino acid residues at the N-terminal or C-terminal are deleted insofar as their characteristic activities (for example, toxicity and agglutinating property lower than those of the wildtype amyloid protein) are not inactivated.

As described in the foregoing, the variant amyloid protein of the first invention includes the natural variant amyloid proteins Aβ1-40 (Δ22E), Aβ1-42 (Δ22E), and Aβ1-43 (Δ22E) existing in familial Alzheimer's disease patients and the non-natural variant amyloid proteins Aβ1-40 (Δ21A), Aβ1-42 (Δ21A), Aβ1-43 (Δ21A), Aβ1-40 (Δ23D), Aβ1-42 (Δ23D), and Aβ1-43 (Δ23D) having the function identical to that of the natural proteins. Also, as used herein, the wording "non-natural" means that the protein has not been isolated and identified from a patient, and there is a chance that existence of a natural amyloid protein from which Ala at position 21 or Asp at position 23 is deleted will be confirmed through examination of a good number of patients.

A second invention is a peptide which is a part of the human variant amyloid protein of the first invention, comprising 5 to 28 amino acids encompassing the site of the deleted amino acid residue in SEQ ID NO 2.

A third invention is a human gene (hereinafter referred to as variant APP gene or deletion variant APP gene in some cases) encoding a variant amyloid precursor protein for the human variant amyloid protein of the first invention.

A fourth invention is an mRNA, which is a transcription product of the human gene of the third invention.

A fifth invention is a cDNA synthesized from the mRNA of the fourth invention, which consists of the base sequence of SEQ ID NO 1 with deletion of the bases at positions 1852 to 1854.

A sixth invention is an antibody which specifically recognizes the human variant amyloid protein of the first invention.

A seventh invention is an antibody which specifically recognizes an oligomeric amyloid protein, which is prepared by using an oligomer of the human variant amyloid protein of claim 1 as an antigen.

An eighth invention is a diagnostic method of amyloid diseases, which comprises detecting an existence of the human amyloid protein of the first invention.

A ninth invention is a diagnostic method of amyloid diseases, which comprises detecting an existence of the human gene of the third invention or the mRNA of the fourth invention.

A tenth invention is a cell expressing the human gene of the third invention.

An eleventh invention is a non-human animal having in vivo the variant amyloid protein of the first invention, and a tissue and a cell derived from the non-human animal.

A twelfth invention is a method for screening a therapeutic agent component for the amyloid diseases, which comprises contacting the cell of the tenth inventionor the non-human animal, the tissue, or the cell derived from the non-human animal of the eleventh invention with a test substance, and measuring the behavior and activity of a human variant amyloid protein in the cell or the tissue.

A thirteenth invention is an anti-amyloid disease drug containing the variant amyloid protein of the first invention.

A fourteenth invention is an anti-amyloid disease drug containing the peptide of the second invention.

A fifteenth invention is an anti-amyloid disease drug containing the cDNA of the fifth invention.

A sixteenth invention is an anti-amyloid disease drug containing the antibody of the sixth invention or the seventh invention.

In this invention, it is possible to compare the deletion variant APP gene and a fragment thereof with the conventional wildtype APP or the reported familial variant APP. As a result, it is expected that new molecular mechanism, action, and effect contributing to clarification of metabolism of amyloid protein will help to elucidate the pathology.

Also, in this invention, it is possible to use a novel gene and animal using the deletion variant APP gene and the fragment thereof for a screening of novel drug and compound.
Also, in this invention, use of 39 amino acids comprising the deletion variant full-length amyloid protein Aβ1-40 (Δ22E), Aβ1-40 (Δ21A), or Aβ1-40 (Δ23D), 41 amino acids comprising the deletion variant full-length amyloid protein Aβ1-42 (Δ22E), Aβ1-42 (Δ21A), or Aβ1-42 (Δ23D), or 42 amino acids comprising the deletion variant full-length amyloid protein Aβ1-43 (Δ22E), Aβ1-43 (Δ21A), or Aβ1-43 (Δ23D) is advantageous for comparing a deletion variant effect thereof with the information of the reported wildtype amyloid protein.

Also, in this invention, pathology of dementia is expected to be induced since the variant has been identified in familial Alzheimer's disease patients, and it is assumed that the portion of Aβ29-40 inside the membrane or the cDNA or the peptide having a partial sequence from which Aβ29-42/43 is excluded is used in order to emphasize on a variant effect. Also, it is possible to conceive a modified matter obtained by inducing a change in hydrophilic property through modification of dissociated group for the purpose of improving cerebral blood vessel barrier or molecular stability.

The variant amyloid protein or the modified matter thereof is a degeneration product of the deletion variant APP, and a considerably reduced protein agglutinating property thereof which had not been known was confirmed.

As used herein, each of "protein" and "peptide" means a molecule consisting of plural amino acid residues which are bounded by peptide binding.

Further, as used herein, "amyloid disease" means diseases, such as Alzheimer's disease and Down's syndrome, in which the amyloid protein participates directly or indirectly as etiology or with which such participation is suspected and diseases in which the amyloid protein is observed in neuron lesion. As used herein, "diagnosis" means a judgment whether or not an examinee suffers from the amyloid disease, a judgment whether or not there is a risk of suffering from the amyloid disease in future, and a judgment whether or not there is a risk of recurrence of the amyloid disease after treatment. Also, the diagnosis includes a measurement of a degree of each of the amyloid disease and the risk.

Other terms and conceptions in this invention will be defined in detail in description of embodiments and examples. Note that the terms are in accordance with IUPAC-IUB Commission on Biochemical Nomenclature or based on meanings of the terms used idiomatically in the art. Also, various technologies used for practicing the invention other than the technologies whose sources are named herein are practicable by person skilled in the art without fail based on known publications and the like. For example, the technologies in the field of the biogenetics and the molecular biology are practicable by the methods disclosed in or the methods disclosed in publications cited in J. Sambrook, E. F. Fritsch & T. Maniatis, "Molecular Cloning: A laboratory manual (2nd edition)", Cold Spring Harbor Laboratory Press, Cold Spring Harbor, New York (1989); D. M. Glover et al. ed., "DNA Cloning", 2nd ed., Vol. 1 to 4, (The Practical Approach Series), IRL Press, Oxford University Press (1995); Ausubel, F. M. et al., Current Protocols in Molecular Biology, John Wiley & Sons, New York, N. Y., 1995; Nihon Seikagakukai ed., "Zoku Seikagaku Jikken Koza 1, Idensi Kenkyuho II", Tokyo Kagaku Dojin (1986); Nihon Seikagakukai ed., "Shin Seikagaku Jikken Koza 2, Kakusan III (Kumikae DNA Gijutsu)", Tokyo Kagaku Dojin (1992); R. Wu ed., "Methods in Enzymology", Vol. 68 (Recombinant DNA), Academic Press, New York (1980); R. Wu et al. ed., "Methods in Enzymology" Vol. 100 (Recombinant DNA, Part B) & 101 (Recombinant DNA, Part C), Academic Press, New York (1983); R. Wu et al. ed., "Methods in Enzymology" Vol. 153 (Recombinant DNA, Part D), 154 (Recombinant DNA, Part E) & 155 (Recombinant DNA, Part F), Academic Press, New York (1987); and the like or by methods substantially the same as those described above and modified methods thereof.

### Brief Description of the Drawings

Fig. 1 is a result of mass analysis of a variant amyloid protein secreted from a variant amyloid precursor protein.
Fig. 2 is a result of a dot test conduced for evaluating antigenicity of variant amyloid protein.
Fig. 3 is a result of a dot test conducted for evaluating antigenicity of the variant amyloid protein on the variant amyloid protein and a wildtype amyloid protein.
Fig. 4 is a result of immune precipitation conducted for evaluating specificity of an anti-variant amyloid protein antibody.
Fig. 5 is a result of immunocytochemistry with the anti-variant amyloid protein antibody.
Fig. 6 is a result of a fluorescent immune tissue double staining conducted for examining a relationship between a variant amyloid protein using the anti-variant amyloid protein antibody and a neuroimmune reaction.

### Best Mode for Carrying out the Invention

It is possible to isolate and generate a variant amyloid proteins of this invention as a natural protein Aβ1-40 (Δ22E), Aβ1-42 (Δ22E), or Aβ1-43 (Δ22E) from a cell of a familial Alzheimer's disease patient, for example. It is possible to obtain non-natural variant amyloid proteins Aβ1-40 (Δ21A), Aβ1-42 (Δ21A), Aβ1-43 (Δ21A), Aβ1-40 (Δ23D), Aβ1-42 (Δ23D), and Aβ1-43 (Δ23D) also by a known peptide synthesizing method. Since materials for the synthesis and methods used in steps performed in the synthesis are well known, person skilled in the art can produce the desired variant amyloid protein as required by appropriately performing synthesis, isolation, purification, and the like. Also, it is possible to produce the variant amyloid proteins of this invention by a gene recombinant technology using known hosts such as *E. coli,* yeast, bacillus subtilis, an insect cell, an animal cell, and a plant cell. A chemical synthesis may be conducted in accordance with Examples described later in this specification, for example, but it is possible to employ any method insofar as a desired gene and peptide or a compound thereof are obtained by the method. For example, it is possible to combine well-known methods such as the reaction for producing Boc (t-butyloxycarbonyl), the DMSO oxidization, the alkali reaction, the acid reaction, the epoxidation reaction, the silica gel column chromatography, the alkylation reaction, the saponification reaction, the heating reaction, the decarboxylation reaction, the condensation reaction, the reverse phase high performance liquid chromatography, and the like. Also, there may be performed a method wherein the amino acid residues constituting the variant amyloid protein are sequentially reacted to evaluate efficiency and reaction product purity as required. Further, the synthesized peptide may be altered at an ordinary temperature, a high temperature, by freezing and thawing, or the like for a desired period of time. More specifically, the method may be performed in accordance with Merrifield, R. B. J. Solid phase peptide synthesis I. The synthesis of tetrapeptide. J. Amer, Chem. Soc. 85, 2149-2154, 1963; Fmoc Solid Phase Peptide Synthesis, A Practical Approach. Chan, W. C. and White, P. D., Oxford University Press, 2000.

The variant amyloid proteins of this invention may include a modification for promoting synthesis and purification, modification for promoting physical and chemical stability, modification for activating stability and instability to in vivo metabolism, conditioning, and the like, and control modification for causing an increase and a reduction in transorgan delivery efficiency including cerebral blood vessel barrier transit. Examples of the control modification include a sequence consisting of 11 amino acids of Tyr-Gly-Arg-Lys-Lys-Arg-Arg-Gln-Arg-Arg-Arg (SEQ ID NO 4) (Schwarze, S. R., Ho, A., Vocero-Akbani, A. & Dowdy, S. F. In vivo protein transduction: Delivery of a biologically active protein into the mouse, Science 285: 1569-1572). By including the control sequence which is linked by the peptide binding at the N-terminal, it is possible to facilitate transit of the variant amyloid protein through the blood cerebral barrier to reach a target site of brain at an improved efficiency.

Other examples of the modification of the variant amyloid proteins of this invention include transfer RNA-mediated addition of amino acid to protein, such as acetylation, acylation, ADP-ribosylation, amidation, flavin covalent binding, covalent binding of hem portion, covalent binding of nucleotide or nucleotide derivative, covalent binding of lipid or lipid derivative, covalent binding of phosphatidylinositol, crosslinking, cyclization, disulfide binding, demethylation, crosslinking covalent binding formation, cystine formation, pyrogultamate formation, formylation, gamma-carboxylation, glycosylation, GPI anchor formation, hydroxylation, iodination, methylation, myristoylation, oxidation, protein hydrolysis processing, phosphorylation, prenylation, racemization, lipid binding, sulfation, selenoylation, and arginylation; ubiquination; and the like.

Further, it is technically easy to add, change, and convert a structure for the purpose of facilitating detection or purification of the variant amyloid protein of this invention or the antibody prepared by using the variant amyloid protein as an immunogen or for the purpose of adding another function, and such products are encompassed by the scope of this invention. Further, an immune globulin Fc fragment such as FLAG-tag, β galactosidase, alkaliphosphatase, and IgG and products such as GFP obtained by a biogenetical method are encompassed by the scope of this invention as the modification addition.

It is possible to create the antibody of the sixth invention by selecting the variant amyloid protein of this invention, its modified matter, or its degenerated matter (partial peptide or the like) and by using the selected matter as an antigen. The partial peptide comprises 20 or less amino acid residues, preferably lesser amino acid residues such as 5 amino acid residues, for example. The antigens may be used in combination for creating the antibody. The antigen is not necessarily the variant amyloid protein of this invention, the modified matter, or the degenerated matter as it is and may be a protein having a primary sequence near the deletion variant site of the variant APP exposed to outside the conformation.

Also, in order to create the antibody immunospecific to the variant amyloid protein of this invention, it is preferable to use a compound containing the above-described amino acid sequence of the structure near the deletion variant. Such antibodies are not particularly limited insofar as they are bounded immunologically to the site or recognize the site. The binding or capability of the recognition of the antibody is decided based on a known antigen antibody reaction. As used herein, "immunospecific" means that affinity to the compound is substantially larger than that to the related proteins or compounds in the art.

Production of the antibody is practiced by performing immune induction having humoral response or cellular response to the antigen by using, as the antigen, the variant amyloid protein of this invention, the modified matter thereof, or the degeneration matter thereof alone or in a state of being bounded to a carrier in the presence or absence of an adjuvant.

It is possible to create the antibody of the seventh invention by creating an oligomer from a peptide obtained by organic synthesis of the variant amyloid protein and then using the oligomeric variant amyloid protein as the antigen. This antibody has novel and useful characteristics that it does not recognize both of variant and wildtype monomeric amyloid proteins such as fibrillary amyloid protein forming senile plaque but specifically recognizes the oligomeric wildtype and variant amyloid proteins.

More specifically, creation of the antibody is implemented by immunizing a mouse by injecting a suspension of the amyloid protein with Freund complete adjuvant, followed by injecting a suspension of the amyloid protein with Freund incomplete adjuvant after a month, and then repeating the same immunization operation after 7 days. Also, it is possible to create the antibody by immunity induction through immune stimulation of a lymph cell or a precursor cell thereof under the cultural condition. The carrier is not particularly limited insofar as it does not cause adverse effect on the host, and examples thereof include, but not limited to, a cellulose, a normal saline, buffering normal saline, dextrose, water, glycerol, ethanol, polymerized amino acid, albumin, a mixture thereof, and the like. As the animal to be immunized, a mouse, a rat, a rabbit, a goat, a horse, a cow, and the like are suitably used. A polychlonal antibody is obtainable as a serum by a known method or by an antibody recovery method from the serum. A preferable method may be an immune affinity chromatography.

Production of a monochlonal antibody is performed in such a manner that a tissue (of spleen or lymph node, for example) or a cultured cell containing antibody activity is recovered from the immunity-induced animal, and then introducing a transformant into a known permanently proliferative cell (myeloma strain such as P3X63Ag8, for example). For example, a hybridoma created from the antibody producing cell and the permanently proliferative cell is subjected to cloning, and then a hybridoma producing the antibody specifically recognizing the variant amyloid protein according to this invention is selected to collect the antibody from a culture medium of the hybridoma. Practical examples include the methods disclosed in the hybridoma method (Kohler G. and Milstein C. (1975) Nature 256, 495-497, the trioma method (Kozbor et al. Immunology Today (1983) 4:72), and the EBV method (Cole et al. Monoclonal antibodies and cancer therapy, Alan R. Liss, Inc., (1985): 77-96), and various other methods.

It is possible to use the antibody for identification, detection, and quantification of the variant amyloid proteins of this invention or for preparation and purification of the variant amyloid proteins through the affinity chromatography and the like. It is possible to modify the antibody into a human antibody by employing a known method.

More specifically, the antibody showing activity of increasing the activity of the variant amyloid protein of this invention among specific antibodies of the variant amyloid proteins of this invention is useful as a calibrator for a compound to be subjected to screening of an amyloid protein generation inhibitory drug or as screening means. Examples of assays using the antibodies for the variant amyloid proteins of this invention include the radioimmunoassay, the competitive binding assay, the high performance liquid chromatography, the western blot analysis, the ELISA assay, and a combination thereof.

The eighth invention and the ninth invention are amyloid disease diagnostic methods characterized by measurement of an expression product of the human variant amyloid protein gene of the first invention. That is, the variant amyloid protein of this invention is a causative protein isolated and identified from the patient with familial Alzheimer's disease, and it is possible to diagnose the amyloid diseases such as the Alzheimer's disease through measurement of abundance of the variant amyloid protein. Further, in the case where the variant amyloid protein of this invention is administered exogenously, it is possible to measure abundance thereof.

In the case where an mRNA is the expression product, it is possible to conduct the measurement by performing quantitative probe hybridization or by using a micro array. As a hybridization method using a standard DNA probe, it is possible to employ the allele-specific oligonucleotide probe method, the oligonucleotide ligation assay, the invader method, and like known methods. Also, it is possible to measure the mRNA amount by a quantitative RT-PCR using an mRNA isolated from an examinee as a template.

Further, in the case where the variant amyloid protein is the gene expression product, it is preferable to use the antibody of the sixth invention. Particularly, use of a labeled antibody makes it possible to achieve detection with convenience and high accuracy. An enzyme, radioisotope, or a fluorochrome may be used for the labeling. The enzyme is not particularly limited insofar as it satisfies conditions such as having a large turnover number, being stable when bounded to the antibody, and specifically staining the matrix, and those ordinarily used for EIA, such as peroxidase, β-galactosidase, alkaliphosphatase, glucose oxidase, acetylcholinesterase, glucose-6-phosphorylated dehydrogenase, and the like may be used as the enzyme. Also, an enzyme inhibitor, coenzyme, and the like may be used. Binding of the enzyme to the antibody may be performed by employing a known method using a crosslinking agent such as a maleimide compound or the like. As the matrix, a known substance may be used depending on the type of the enzyme to be used. For example, 3,3'5,5'-tetramethylbenzydine is used in the case of using peroxidase as the enzyme, or, paranitrophenol or the like is used in the case of using alkaliphosphatase as the enzyme. As the radioisotope, those used in ordinary RIA, such as ¹²⁵I and ³H, may be used. As the fluorochrome, those used in ordinary fluorescent antibody methods, such as fluorescence isothiocyanate (FITC) and tetramethylrhodamine isothiocyanate (TRITC) may be used. In the case of using the enzyme, a matrix capable of coloring by degeneration through an enzymatic effect is added; enzyme activity is determined by optically measuring a degeneration amount of the matrix; the enzyme activity is converted into a bounded antibody amount; and an antibody amount is calculated through comparison with a standard value. In the case of using the radioisotope, a radiation amount generated from the radioactive isotope is measured by a scintillation counter or the like. In the case of using the fluorochrome, an amount of fluorescence is measured by using a measurement device combined with a fluorescence microscope. Also, it is possible to perform the measurement by using a flow cytometer. Further, a sandwich method (the ELISA method when an enzyme is used for labeling) using a primary antibody and a labeled secondary antibody may preferably be used.

Hereinafter, the cell of the tenth invention and the animal of the eleventh invention will be described. It is possible to create the animal or the cell by a method of injecting the variant amyloid protein solution to an animal, a method of administering a substance obtained by a metal colloid and the variant amyloid protein to an animal, a method of introducing the variant APP gene of the second invention or the cDNA thereof into an animal or a cell in accordance with a gene therapy using a retrovirus vector or an adenovirus vector, a calcium phosphate method, a method of using a liposome or a red blood cell ghost, an electropolation method, a method of introducing the variant amyloid protein or the peptide thereof into a cell by a microinjection method using a glass pipette, and the like.

Further, it is possible to create the animal and the cell in accordance with a known transgenic animal creation method (Proc. Natl. Acad. Scl. USA 77; 7380-7384, 1980, for example). More specifically, it is possible to create a desired transgenic animal by introducing the variant APP gene of the second invention into a totipotency cell of a non-human animal (preferably a mouse or a rat) to generate a solid matter from the cell and then selecting the solid matter in which the variant APP gene is incorporated into a genome of a somatic cell. As the method of gene introduction into the totipotency cell, a physical injection of an exogenous gene DNA (microinjection method) is optimal in view of a production efficiency of the solid transgenic animal and a transfer efficiency of the introduced gene to the next generation. A fertilized egg into which the gene was injected is transplanted in a fallopian tube of a foster parent, and then an animal born as an individual is brought up beside the foster parent, followed by extraction of a DNA from a part of the body (tip of tail or the like). After that, southern blot analysis or a PCR assay is performed so as to confirm existence of an exogenous gene in the extracted DNA. A primary heterozygous animal in which the exogenous gene is introduced into one of diploid chromosomes is obtained as described above, and it is possible to obtain a homozygous animal by mating the heterozygous animals. The transgenic animal of this invention includes the primary heterozygous animal, the homozygous animal obtained by mating the heterozygous animals, descendants thereof, and embryos thereof.

The twelfth invention is the method for probing a therapeutic agent component for the amyloid diseases using the cell or the animal. More specifically, a candidate substance is brought into contact with the animal or the cell, and an amount of an expression product (mRNA or variant amyloid protein) of a variant APP gene expressing in the animal tissue (central nervous system tissue) in the cell by the methods same as those of the diagnostic methods of the eighth and ninth inventions. The candidate substance capable of reducing the expression product amount is used as the therapeutic agent component. Also, a substance influencing on the activity of the variant amyloid protein expressing in the cell or the animal tissue is determined as the therapeutic agent component.

The candidate substance to be used for the screening method includes an organic or inorganic compound (particularly low molecular compound), a protein, a peptide, and the like, for example. Of these substances, functions and structures may be known or unknown. Also, combinatorial chemical library is effective means as a candidate substance group for specifying the desired substance efficiently. The combinatorial chemical library is a collection of chemical compositions generated by combining various chemical building blocks of reagents and the like by a chemical synthesis or a biological synthesis. For example, a linear combinatorial chemical library such as a peptide library is formed by combining a set of building blocks (amino acids) with a length of a given compound (the size of the peptide) by all possible methods. It is possible to synthesize various chemical compositions through the combinatorial mixing of chemical building blocks. For example, systematic combinatorial mixing of 100 commutative chemical building blocks results in 100 million tetrameric compounds or 10 billion pentameric compounds (see Gallop et al., (1994) 37(9): 1233-1250, for example). Preparation and screening of combinatorial chemical library are well-known in the art (see United States Patent Nos. 6,004,617 and 5,985,365, for example). Also, it is possible to use various commercially available libraries.

The thirteenth to sixteenth inventions are anti-amyloid disease drugs each containing the variant amyloid protein, the peptide, the cDNA, or the antibody of this invention. Each of the drugs controls an amyloid protein production amount or a cerebral accumulation amount thereof to prevent and treat the diseases as well as to ameliorate symptoms. More specifically, the antibodies of the sixth invention and the seventh invention are provided for solving problems of adverse reaction in the conventional indirect immune therapy. The variant amyloid protein, the peptide, or the cDNA functions as an antigen molecule for producing an antibody effective for inhibiting or suppressing polymerization of the wildtype amyloid protein in vivo.

Each of the components is formulated by known method and means into the form which is capable of being introduced into a living body or cell. In the case of the cDNA, a drug form may be such that an expression vector into which the cDNA is integrated in integrated into hollow nanoparticles presenting biorecognition molecules or a known virus vector (retrovirus, adenovirus, adeno-associated virus), for example. By introducing such drug into a living body by a gene therapy method, it is possible to express the variant amyloid protein in living cells. It is also possible to adopt a gene therapy method of injecting a solution of the cDNA into the muscle. Further, in order to formulate the variant amyloid protein, the peptide, or the antibody into the form introducible into a living body, it is possible to blend the variant amyloid protein, the peptide, or the antibody with a carrier solution which does not change structures and functions thereof and is pharmacologically acceptable. It is also possible to employ a method wherein an organ or a cell taken out, of a living body is subjected to a treatment with the protein or the peptide in vitro to induce a desired character and then the organ or the cell is returned to the living body. Also, a method wherein a cDNA expression vector is introduced into an organ or a cell taken out of a living body and then the organ or the cell is returned to the living body may be employed as a mode of the therapy. In such case, the microinjection method may be employed for the introduction into the cell, for example. Also, an intracellular introduction method using lipid (BioPORTER (Gene Therapy Systems, USA), Chariot (Active Motif, USA), and the like) may be employed. Further, each of the anti-amyloid disease drugs of this invention may be formulated into a proper form that enhances transport efficiency thereof to the cerebral tissue.

The variant amyloid protein, the peptide, the cDNA, or the antibody, which are the effective ingredients, may be used alone or in combination with a modified matter thereof or a degenerated matter thereof. Further, the variant amyloid protein, the peptide, the cDNA, or the antibody may be used concomitantly with a compound that is advantageous for the therapy. A preferable mode of a systemic administration of the drug of this invention is injection, particularly intravenous injection. It is also possible to use another injection pathway such as subcutaneous injection, intramuscular injection, or intraperitoneal injection. Another means for the systemic administration is a transmucosal or transdermal administration using a penetrating agent such as a bile acid salt and adrenal acid or other surfactants. Further, when an enteric formulation or a capsule formulation is successfully made, an oral administration is possible. The administration of the drug compositions may be topical, and a form may be an ointment, a paste, a gel, or the like.

### Examples

Hereinafter, this invention will be described in particulars based on but not limited to the following Examples.

### Example 1

### Determination of Deletion Variant APP Base Sequence

Genome DNA was extracted from blood of a familial Alzheimer's disease patient, and total base sequences each encoding a cDNA of each of presenilin 1 (PSEN1), presenilin 2 (PSEN2), and APP, which are known to be causative genes, were subjected to PCR amplification by using the obtained DNA as a matrix and then analyzed with the use of PRISM model 310 sequencer manufactured by ABI (Perkin-Elmer, CA). As a result, a variant was found in an amyloid protein coding region (SEQ ID NO 1) of the wildtype APP gene cDNA (deletion of three bases gaa at positions 1852 to 1854 of SEQ ID NO 1).

### Example 2

### Determination of Structure of Amyloid Protein Synthesized from Deletion Variant APP Gene cDNA

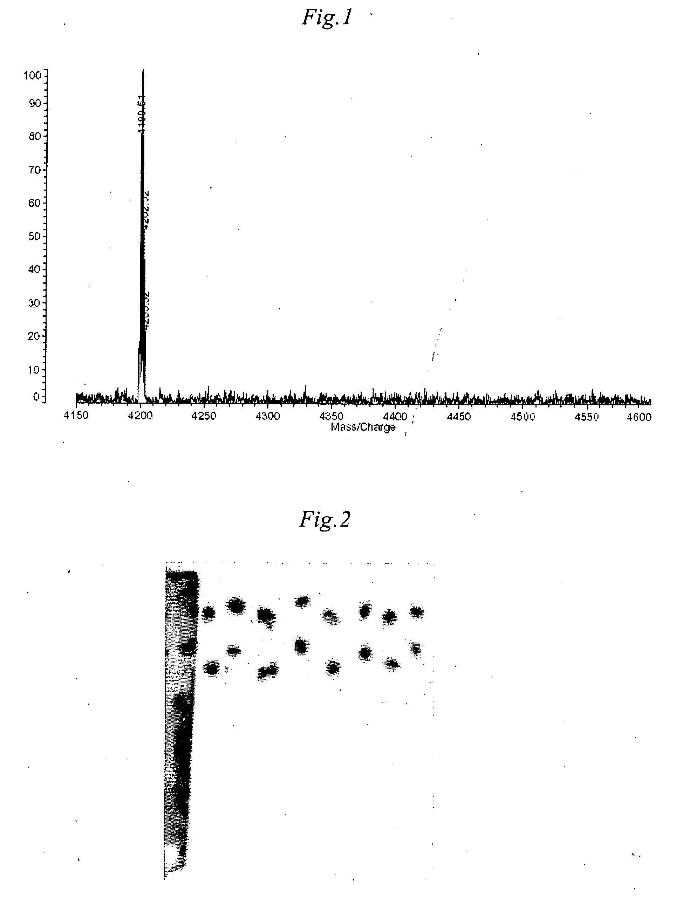

Since the variant APP gene cDNA obtained in Example 1 had the variant inside the amyloid protein, a β secretase or γ secretase cleavage site was examined. Then, an expression vector of the variant APP gene cDNA was introduced into human cultured cell HEK cells known to express APP as well as to produce and secrete amyloid protein. The expression was left to occur for 2 days to obtain a culture supernatant, and an immunoprecipitated fraction obtained by using an anti-amyloid protein antibody was subjected to a mass analysis with the use of AXIMA-CFR manufactured by Shimadzu Corporation. The result is shown in Fig. 1.
As is apparent from Fig. 1, an experimental observation value obtained as a molecular weight of Aβ1-40 (ΔE) which is the amyloid protein secreted from the variant APP gene cDNA expressed in the human cultured cell HEK cells was 4200.51. This molecular weight was almost the same as that of a theoretical calculation value 4200.69 of a molecular weight in the case of a deletion of Glu at position 21 of Aβ1-40. That is, it was demonstrated that the deletion variant amyloid protein is produced and secreted in a state where the conventional β secretase and γ secretase cleavage sites are retained in this deletion variant APP. More specifically, it was demonstrated that the variant amyloid protein is Aβ1-40 wherein an amino terminal starts with Asp and terminates with Val with Glu at position 22 being deleted.

### Example 3

### Quantification of Amyloid Protein Synthesized from Deletion Variant APP

It was demonstrated that the deletion variant APP expressed in the human cultured cell HEK cells produces and secretes two types of variant amyloid proteins similar to the wildtype APP as shown in Tale 1.

**Table 1**

| | Aβ42 (pg/ml) | Aβ40 (pg/ml) |
|---|---|---|
| Control | 0 | 0 |
| Wildtype APP | 26 | 200 |
| Variant APP | 24 | 190 |

Note that a vacant expression vector dissolved into a normal saline was used as the control in Table 1. Also, Aβ42 represents Aβ1-42 and Aβ1-43 or Aβ1-42 (ΔE) and Aβ1-43 (ΔE), and Aβ40 represents Aβ1-40 or Aβ1-40 (ΔE).

### Example 4

### Toxicity of Variant Amyloid Protein

The variant amyloid protein used in Example 4 was an organically synthesized peptide that has an amino acid sequence and a mass same as those of the variant amyloid protein secreted from the cells according to amino acid sequence and mass analyses. Through examination of neuron toxicity with the use of the synthetic peptide, it was demonstrated that the synthetic peptide has a lower toxicity than the conventional wildtype amyloid protein. The result is shown in Table 2

**Table 2**

| | Control | Wildtype Aβ | Variant 90 Aβ |
|---|---|---|---|
| Survival Rate (%) | 100 | 40 | 90 |

In Table 2, the wildtype Aβ or the variant Aβ represents Aβ1-40 or Aβ1-40 (ΔE), and the control represents Aβ1-40 having a reverse amino acid sequence of an amino acid sequence of the wildtype Aβ1-40.

### Example 5

### Agglutinating Property of Variant Amyloid Protein

The variant amyloid protein used in Example 5 was an organically synthesized peptide that has an amino acid sequence and a mass same as those of the variant amyloid protein secreted from the cell according to amino acid sequence and mass analyses. Through examination of protein agglutinating property by using the peptide by thioflavine T binding ability, it was demonstrated that the agglutinating activity of the variant amyloid protein of this invention is considerably lower than the conventional wildtype amyloid protein. The result is shown in Table 3.

**Table 3**

| | 0 Hr | 12 Hr | 24 Hr |
|---|---|---|---|
| Wildtype Aβ | 0 | 61 | 100 |
| Variant Aβ | 0 | 2 | 2 |

In Table 3, the wildtype Aβ represents Aβ1-42, and the variant Aβ represents Aβ1-42 (ΔE). The agglutinating activity of the wildtype Aβ1-42 under the conditions of 100 micrograms/ml (PBS) at 37°C for 24 hours was set to 100%.

### Example 6

### Antigenicity of Variant Amyloid Protein

The peptide organically synthesized from the variant amyloid protein was heated at 37°C for 24 hours to create an oligomer. Identification of the oligomer was conducted by confirming: detection of a large amyloid complex (dimeric, trimeric, or more) by Western blot; absence of amyloid fiber proved by electronic microscopic observation; and thioflavine binding reaction negative.
An antibody having the oligomeric variant amyloid protein as an antigen was created. For this purpose, a suspension of a peptide dissolved into PBS and Freund complete adjuvant solution was immune-injected into a mouse. After a month, the mouse was immunized with a suspension containing Freund incomplete adjuvant in place of Freund complete adjuvant, and the same immunization operation was repeated after 7 days. Blood in an amount of 100 microlitters was collected from the tail vein of the mouse, and reactivity thereof was confirmed.
A plurality of PVDF membrane strips spotted with the antigen peptide was prepared. After drying the PVDF membrane strips, they were blocked with PBS containing a 20% cow serum. Then, the membrane strips were separately placed in small reaction boxes to be reacted with 1/150 diluted mouse serums (No. 1 to 8) (Fig. 2). The membrane strips were subjected to a reaction with an avidin-biotin complex by using biotinylated labeled antimouse IgG goat antibody as a secondary antibody. Detection was performed by observing antibody titers through DAB coloring. The leftmost membrane strip is one example of a reaction using as a control a rabbit antibody capable of recognizing an N-terminal margin.
Referring to Fig. 2, membrane strips shown in the dot test were spotted as being shifted in a vertical direction from one another in order to distinguish the one from another. The membrane strips were dotted with 10 ng, 1 ng, 0.1 ng, and 0.01 ng, PBS and albumin in this order from the top. It was revealed that the antiserum of 1/150 dilution requires at least 1 ng of antigen and is not detected when the antigen amount is less than 1 ng. Further, there was no reaction with bovine serum albumin (BSA). The important fact is that the antibody capable of recognizing the oligomer peptide was identified in all the mouse serums of the 8 test mice without exception, and that the high and reproducible antigenicity of the oligomeric variant amyloid protein was confirmed.

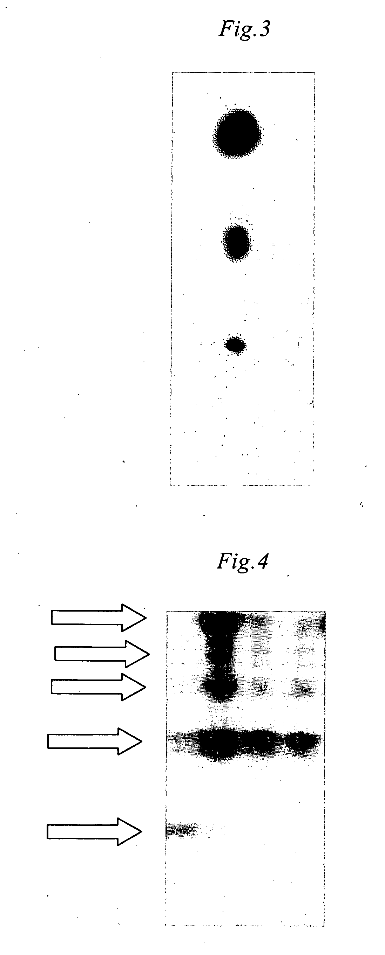

Further, it was confirmed that an anti-variant amyloid protein antibody creased by using the variant amyloid protein as an antigen reacts not only with the variant amyloid protein but also with the wildtype amyloid protein (Fig. 3). Referring to Fig. 3, the test was conducted in the same manner as described above except for using the wildtype amyloid protein in place of the variant amyloid protein. The membrane strip was dotted with 10 ng, 1 ng, 0.1 ng, and 0.01 ng of the wildtype amyloid protein, PBS, and albumin in this order from the top.

### Example 7

### Specificity of Anti-Variant Amyloid Protein Antibody

Specificity of the anti-variant amyloid protein antibody was confirmed in such a manner that the variant amyloid protein oligomer was immunoprecipitated by using the obtained antiserum and then detected by using another ordinary amyloid antibody by western blot.
The leftmost membrane strip in the immunoprecipitation test shown in Fig. 4 is one example of a reaction using as a control a rabbit antibody capable of recognizing an N-terminal margin and detection with the use of the same antibody. The arrows indicate electrophoresis patterns of the amyloid proteins, and the monomeric form (molecular weight: 4 kDa), the dimeric form (molecular weight: 8 kDa), the trimeric form (molecular weight: 12 kDa), the tetrameric form (molecular weight: 16 kDa), and the pentameric or larger form (molecular weight: 20 kDa) are shown in this order from the bottom to the top. It was revealed that mouse serums examined herein (Nos. 2, 3, and 4) recognize and bounded to the dimeric or larger oligomers. The ordinary amyloid antibody recognizes monomeric form, but the anti-variant amyloid protein antibody does not recognize the monomeric form.

Next, oligomer antibody activity of the anti-variant amyloid protein antibody was quantitatively measured (Table 4). In this measurement, 100 ng, 10 ng, and 1 ng of the variant amyloid protein oligomer, PBS, or cow albumin were bounded to a 96-hole plastic plate and then blocked by a 20% cow serum PBS to test 1/450 diluted mouse serums (Nos. 2, 3, and 4). A peroxidase-labeled antimouse IgG goat antibody was used as a secondary antibody.

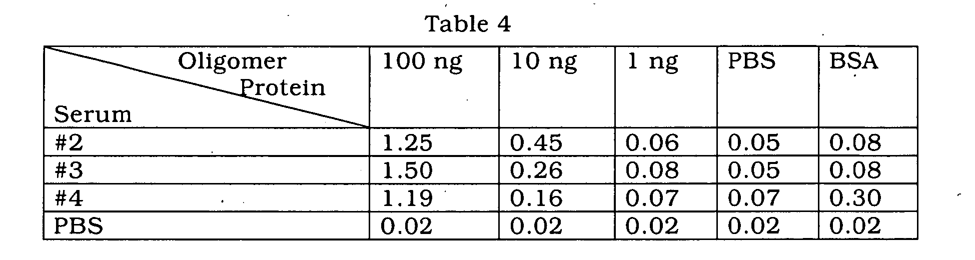

It is apparent from Table 4 that the anti-variant amyloid protein oligomer antibody does not react with BSA nor the monomeric amyloid protein but reacts specifically with the oligomeric amyloid protein.

### Example 8

### Immunohistochemistry with Anti-Variant Amyloid Protein Antibody

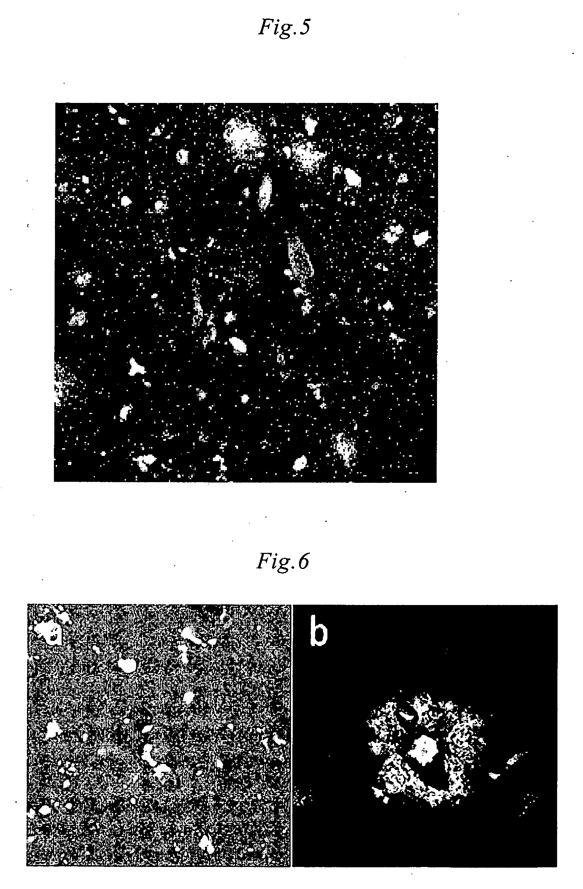

Specificity of the anti-variant amyloid protein antibody was examined through fluorescent antibody staining using a formalin-fixed Alzheimer's disease cerebral tissue section (Fig. 5). The anti-variant amyloid protein antibody was reacted at 4°C overnight as a primary antibody, and then the cerebral tissue section wash washed with PBS containing a 0.05% Tween 20, followed by reaction with a Texas Red-labeled antimouse IgG antibody which was used as a secondary antibody for 2 hours, and then washed again to be encapsulated with an encapsulating agent containing an fluorescent antiphotobleaching agent. Observation was conducted by using a confocal laser scanning microscope (Axiovert 100; product of Zeiss, Germany).
The result is as shown in Fig. 5, and the stainability was such that a dot-like distribution in neuron was observed, which coincides with reported publications of the oligomeric amyloid protein (Non-Patent Document 8; R. H. Takahashi, C. G. Almeida, P. F. Kearey, F. Yu, M. T. Lin, T. A. Milner, G. K. Gouras, Oligomerization of Alzheimer's amyloid within processes and synapses of cultured neurons and brain, (2004) J. Neurosci. 24: 3592-3599; R. Kayed, E. Head, J. L. Thompson, T. M. Mclntire, S. C. Milton, C. W. Cotman, C. G. Glabe, Common structure of soluble amyloid oligomers implies common mechanism of pathogenesis, (2003) Science 300: 486-489).
More specifically, it was confirmed that the anti-variant amyloid protein antibody created by using the oligomeric amyloid protein as the antibody recognizes the oligomeric amyloid protein but does not recognize senile plaque that the fibrous amyloid protein forms.

### Example 9

### Anti-Variant Amyloid Protein Antibody and Neuroimmune Reaction

Antibodies induced by the conventional vaccine therapy (D. Schenk, R. Barbour, W. Dunn, et al. Immunization with amyloid-beta attenuates Alzheimer-disease-like pathology in the PDAPP mouse, (1999) Nature 400: 173-177) use the wildtype amyloid protein (Abeta₁₋₄₂) as the antigen and stain senile plaque ( C. Hock, U. Konietzko, A. Papassotiropoulos, et al., Generation of antibodies specific for beta-amyloid by vaccination of patients with Alzheimerdisease, (2002) Nat Med 8: 1270-1275). Further, it has been reported that adverse effect of an encephalitis reaction had been caused by the antibody (J. A. Nicoll, D. Wilkinson, C. Holmes, P. Steart, H. Markham, R. O. Weller, Neuropathology of human Alzheimer's disease following immunization with amyloid beta peptide: a case report, (2003) Nat Med 9: 448-452; J. M. Orgogozo, S. Gilman, J. F. Dartigues, B. Laurent, M. Puel, L. C. Kirby, P. Jouanny, B. Dubois, L. Eisner, S. Flitman, B. F. Michel, M. Boada, A. Frank, & C. Hock, Subacute meningoencephalitis in a subset of patients with AD after abeta42 immunization, (2003) Neurology 61: 46-54).
In order to compare a complement molecule (C3d) activated as an inflammatory reaction marker with the stains with the anti-variant amyloid protein antibody, an anti-variant amyloid protein mouse antibody and an anti-C3d rabbit antibody were mixed to be used as the primary antibodies and then reacted at 4°C overnight, and then the cerebral tissue section was washed with PBS containing a 0.05% Tween 20, followed by reaction with a mixture solution of a Texas Red-labeled antimouse IgG antibody and an FITC-labeled antirabbit IgG antibody, which were used as the secondary antibodies, for 2 hours, and then washed again to be encapsulated with an encapsulating agent containing a fluorescent antiphotobleaching agent. Observation was conducted by using a confocal laser scanning microscope (Axiovert 100; product of Zeiss, Germany).
The result is as shown in Fig. 6. Shown in Fig. 6 are double stain images of the same section. Shown in Fig. 6(a) is the staining image of the anti-variant amyloid protein mouse antibody, and shown in Fig. 6(b) is the staining image of the anti-C3d rabbit antibody, which were detected as different fluorescent images. As is coinciding with the conventional report (P. Eikelenboom, F. C. Stam, Immunoglobulins and complement factors in senile plaques, (1982) Acta Neuropathol 57: 239-242; P. Eikelenboom, C. E. Hack, J. M. Rozemuller, F. C. Stam, Complement activation in amyloid plaques in Alzheimer's dementia, (1989) Virchows Arch 56: 259-262; P. Eikelenboom, J. M. Rozemuller, F. L. van Muiswnkel, Inflammation and Alzheimer's disease: relationships between pathogenic mechanisms and clinical expression, (1998) Exp Neurol 154: 89-98; P. L. McGeer, E. G. McGeer, inflammation, autotoxicity and Alzheimer disease, (2001) Neurobiology of Aging 22: 799-809; P. Eikelenboom, A. J. M. Rozemuller, J. J. M. Hoozemans, R. Veerhuis, & W. A. van Gool, Neuroinflammation and Alzheimer Disease: Clinical and Therapeutic Implications, Alzheimer Disease & Associated Disorders, (2000) 14, Suppl. 1: S54-S61), C3d which is an activated neuroimmune complement reaction marker has a distribution coinciding with senile plaque. However, Alzheimer's disease cerebral tissue localization of C3d did not coincide with the oligomeric amyloid protein localization which is the anti-variant amyloid protein antibody staining image.

### Industrial Applicability

This invention provides a novel variant amyloid protein and its gene relating to pathology of amyloid diseases such as Alzheimer's disease, and provisions of a novel drug composition and a diagnostic measure utilizing the characteristic are useful in the fields of clinical and basic studies particularly of the amyloid diseases such as Alzheimer's disease.

## Claims

1. A human variant amyloid protein, which consists of the following amino acid sequence taken from SEQ ID NO: 2:
(1) the 39 amino acids from position 597 to position 636 with the deletion of glutamic acid at position 618;
(2) the 41 amino acids from position 597 to position 638 with the deletion of glutamic acid at position 618;
(3) the 42 amino acids from position 597 to position 639 with the deletion of glutamic acid at position 618;
(4) the 39 amino acids from position 597 to position 636 with the deletion of alanine at position 617;
(5) the 41 amino acids from position 597 to position 638 with the deletion of alanine at position 617;
(6) the 42 amino acids from position 597 to position 639 with the deletion of alanine at position 617;
(7) the 39 amino acids from position 597 to position 636 with the deletion of aspartic acid at position 619;
(8) the 41 amino acids from position 597 to position 638 with the deletion of aspartic acid at position 619;
(9) the 42 amino acids from position 597 to position 639 with the deletion of aspartic acid at position 619.

2. A peptide which is a part of the human variant amyloid protein of claim 1, comprising 5 to 28 amino acids encompassing the site of the deleted amino acid residue in SEQ ID NO. 2.

3. A human gene encoding a variant amyloid precursor protein for the human variant amyloid protein of claim 1.

4. An mRNA, which is a transcription product of the human gene of claim 3.

5. A cDNA synthesized from the mRNA of claim 4, which consists of the base sequence of SEQ ID NO. 1 with deletion of the bases at positions 1852 to 1854.

6. An antibody which specifically recognizes the human variant amyloid protein of claim 1.

7. An antibody which specifically recognizes an oligomeric amyloid protein, which is prepared by using an oligomer of the human variant amyloid protein of claim 1 as an antigen.

8. A diagnostic method of amyloid diseases, which comprises detecting an existence of the human amyloid protein of claim 1.

9. A diagnostic method of amyloid diseases, which comprises detecting an existence of the human gene of claim 3 or the mRNA of claim 4.

10. A cell expressing the human gene of claim 3.

11. A non-human animal having in vivo the variant amyloid protein of claim 1 and a tissue and a cell derived from the non-human animal.

12. A method for screening a therapeutic agent component for amyloid diseases, which comprises contacting the cell of claim 10, or the non-human animal, the tissue, or the cell derived from the non-human animal of claim 11 with a test substance, and measuring the behaviour and activity of a human variant amyloid protein in the cell or the tissue.

13. An anti-amyloid disease drug containing the variant amyloid protein of claim 1.

14. An anti-amyloid disease drug containing the peptide of claim 2.

15. An anti-amyloid disease drug containing the cDNA of claim 5.

16. An anti-amyloid disease drug containing the antibody of claim 6 or 7.


SEQUENCE LISTING

<110> MORI, Hiroshi

<120> Mutant amyloid protein

<130> 05F076PCT

<150> JP2004-294292
<151> 2004-10-06

<160> 4

<170> PatentIn version 3.1

<210> 1
<211> 2088
<212> DNA
<213> Homo sapiens

<220>
<221> CDS
<222> (1)..(2088)
<223>

<400> 1
atg ctg ccc ggt ttg gca ctg ctc ctg ctg gcc gcc tgg acg gct cgg 48
Met Leu Pro Gly Leu Ala Leu Leu Leu Leu Ala Ala Trp Thr Ala Arg
1               5                   10                  15

gcg ctg gag gta ccc act gat ggt aat gct ggc ctg ctg gct gaa ccc 96
Ala Leu Glu Val Pro Thr Asp Gly Asn Ala Gly Leu Leu Ala Glu Pro
            20                  25                  30

cag att gcc atg ttc tgt ggc aga ctg aac atg cac atg aat gtc cag 144
Gln Ile Ala Met Phe Cys Gly Arg Leu Asn Met His Met Asn Val Gln
        35                  40                  45

aat ggg aag tgg gat tca gat cca tca ggg acc aaa acc tgc att gat 192
Asn Gly Lys Trp Asp Ser Asp Pro Ser Gly Thr Lys Thr Cys Ile Asp
    50                  55                  60

acc aag gaa ggc atc ctg cag tat tgc caa gaa gtc tac cct gaa ctg 240
Thr Lys Glu Gly Ile Leu Gln Tyr Cys Gln Glu Val Tyr Pro Glu Leu
65                  70                  75                  80

cag atc acc aat gtg gta gaa gcc aac caa cca gtg acc atc cag aac 288
Gln Ile Thr Asn Val Val Glu Ala Asn Gln Pro Val Thr Ile Gln Asn
                85                  90                  95

tgg tgc aag cgg ggc cgc aag cag tgc aag acc cat ccc cac ttt gtg 336
Trp Cys Lys Arg Gly Arg Lys Gln Cys Lys Thr His Pro His Phe Val
            100                 105                 110

att ccc tac cgc tgc tta gtt ggt gag ttt gta agt gat gcc ctt ctc 384
Ile Pro Tyr Arg Cys Leu Val Gly Glu Phe Val Ser Asp Ala Leu Leu
        115                 120                 125

gtt cct gac aag tgc aaa ttc tta cac cag gag agg atg gat gtt tgc 432
Val Pro Asp Lys Cys Lys Phe Leu His Gln Glu Arg Met Asp Val Cys
    130                 135                 140

gaa act cat ctt cac tgg cac acc gtc gcc aaa gag aca tgc agt gag 480
Glu Thr His Leu His Trp His Thr Val Ala Lys Glu Thr Cys Ser Glu
145                 150                 155                 160

aag agt acc aac ttg cat gac tac ggc atg ttg ctg ccc tgc gga att 528
Lys Ser Thr Asn Leu His Asp Tyr Gly Met Leu Leu Pro Cys Gly Ile

                165                 170                 175

gac aag ttc cga ggg gta gag ttt gtg tgt tgc cca ctg gct gaa gaa 576
Asp Lys Phe Arg Gly Val Glu Phe Val Cys Cys Pro Leu Ala Glu Glu
            180                 185                 190

agt gac aat gtg gat tct gct gat gcg gag gag gat gac tcg gat gtc 624
Ser Asp Asn Val Asp Ser Ala Asp Ala Glu Glu Asp Asp Ser Asp Val
        195                 200                 205

tgg tgg ggc gga gca gac aca gac tat gca gat ggg agt gaa gac aaa 672
Trp Trp Gly Gly Ala Asp Thr Asp Tyr Ala Asp Gly Ser Glu Asp Lys
    210                 215                 220

gta gta gaa gta gca gag gag gaa gaa gtg gct gag gtg gaa gaa gaa 720
Val Val Glu Val Ala Glu Glu Glu Glu Val Ala Glu Val Glu Glu Glu
225                 230                 235                 240

gaa gcc gat gat gac gag gac gat gag gat ggt gat gag gta gag gaa 768
Glu Ala Asp Asp Asp Glu Asp Asp Glu Asp Gly Asp Glu Val Glu Glu
                245                 250                 255

gag gct gag gaa ccc tac gaa gaa gcc aca gag aga acc acc agc att 816
Glu Ala Glu Glu Pro Tyr Glu Glu Ala Thr Glu Arg Thr Thr Ser Ile
            260                 265                 270

gcc acc acc acc acc acc acc aca gag tct gtg gaa gag gtg gtt cga 864
Ala Thr Thr Thr Thr Thr Thr Thr Glu Ser Val Glu Glu Val Val Arg
        275                 280                 285

gtt cct aca aca gca gcc agt acc cct gat gcc gtt gac aag tat ctc 912
Val Pro Thr Thr Ala Ala Ser Thr Pro Asp Ala Val Asp Lys Tyr Leu
    290                 295                 300

gag aca cct ggg gat gag aat gaa cat gcc cat ttc cag aaa gcc aaa 960
Glu Thr Pro Gly Asp Glu Asn Glu His Ala His Phe Gln Lys Ala Lys
305                 310                 315                 320

gag agg ctt gag gcc aag cac cga gag aga atg tcc cag gtc atg aga 1008
Glu Arg Leu Glu Ala Lys His Arg Glu Arg Met Ser Gln Val Met Arg
                325                 330                 335

gaa tgg gaa gag gca gaa cgt caa gca aag aac ttg cct aaa gct gat 1056
Glu Trp Glu Glu Ala Glu Arg Gln Ala Lys Asn Leu Pro Lys Ala Asp
            340                 345                 350

aag aag gca gtt atc cag cat ttc cag gag aaa gtg gaa tct ttg gaa 1104
Lys Lys Ala Val Ile Gln His Phe Gln Glu Lys Val Glu Ser Leu Glu
        355                 360                 365

cag gaa gca gcc aac gag aga cag cag ctg gtg gag aca cac atg gcc 1152
Gln Glu Ala Ala Asn Glu Arg Gln Gln Leu Val Glu Thr His Met Ala
    370                 375                 380

aga gtg gaa gcc atg ctc aat gac cgc cgc cgc ctg gcc ctg gag aac 1200
Arg Val Glu Ala Met Leu Asn Asp Arg Arg Arg Leu Ala Leu Glu Asn
385                 390                 395                 400

tac atc acc gct ctg cag gct gtt cct cct cgg cct cgt cac gtg ttc 1248
Tyr Ile Thr Ala Leu Gln Ala Val Pro Pro Arg Pro Arg His Val Phe
                405                 410                 415

aat atg cta aag aag tat gtc cgc gca gaa cag aag gac aga cag cac 1296
Asn Met Leu Lys Lys Tyr Val Arg Ala Glu Gln Lys Asp Arg Gln His
            420                 425                 430

acc cta aag cat ttc gag cat gtg cgc atg gtg gat ccc aag aaa gcc 1344
Thr Leu Lys His Phe Glu His Val Arg Met Val Asp Pro Lys Lys Ala

        435                 440                 445

gct cag atc cgg tcc cag gtt atg aca cac ctc cgt gtg att tat gag 1392
Ala Gln Ile Arg Ser Gln Val Met Thr His Leu Arg Val Ile Tyr Glu
    450                 455                 460

cgc atg aat cag tct ctc tcc ctg ctc tac aac gtg cct gca gtg gcc 1440
Arg Met Asn Gln Ser Leu Ser Leu Leu Tyr Asn Val Pro Ala Val Ala
465                 470                 475                 480

gag gag att cag gat gaa gtt gat gag ctg ctt cag aaa gag caa aac 1488
Glu Glu Ile Gln Asp Glu Val Asp Glu Leu Leu Gln Lys Glu Gln Asn
                485                 490                 495

tat tca gat gac gtc ttg gcc aac atg att agt gaa cca agg atc agt 1536
Tyr Ser Asp Asp Val Leu Ala Asn Met Ile Ser Glu Pro Arg Ile Ser
            500                 505                 510

tac gga aac gat gct ctc atg cca tct ttg acc gaa acg aaa acc acc 1584
Tyr Gly Asn Asp Ala Leu Met Pro Ser Leu Thr Glu Thr Lys Thr Thr
        515                 520                 525

gtg gag ctc ctt ccc gtg aat gga gag ttc agc ctg gac gat ctc cag 1632
Val Glu Leu Leu Pro Val Asn Gly Glu Phe Ser Leu Asp Asp Leu Gln
    530                 535                 540

ccg tgg cat tct ttt ggg gct gac tct gtg cca gcc aac aca gaa aac 1680
Pro Trp His Ser Phe Gly Ala Asp Ser Val Pro Ala Asn Thr Glu Asn
545                 550                 555                 560

gaa gtt gag cct gtt gat gcc cgc cct gct gcc gac cga gga ctg acc 1728
Glu Val Glu Pro Val Asp Ala Arg Pro Ala Ala Asp Arg Gly Leu Thr
                565                 570                 575

act cga cca ggt tct ggg ttg aca aat atc aag acg gag gag atc tct 1776
Thr Arg Pro Gly Ser Gly Leu Thr Asn Ile Lys Thr Glu Glu Ile Ser
            580                 585                 590

gaa gtg aag atg gat gca gaa ttc cga cat gac tca gga tat gaa gtt 1824
Glu Val Lys Met Asp Ala Glu Phe Arg His Asp Ser Gly Tyr Glu Val
        595                 600                 605

cat cat caa aaa ttg gtg ttc ttt gca gaa gat gtg ggt tca aac aaa 1872
His His Gln Lys Leu Val Phe Phe Ala Glu Asp Val Gly Ser Asn Lys
    610                 615                 620

ggt gca atc att gga ctc atg gtg ggc ggt gtt gtc ata gcg aca gtg 1920
Gly Ala Ile Ile Gly Leu Met Val Gly Gly Val Val Ile Ala Thr Val
625                 630                 635                 640

atc gtc atc acc ttg gtg atg ctg aag aag aaa cag tac aca tcc att 1968
Ile Val Ile Thr Leu Val Met Leu Lys Lys Lys Gln Tyr Thr Ser Ile
                645                 650                 655

cat cat ggt gtg gtg gag gtt gac gcc gct gtc acc cca gag gag cgc 2016
His His Gly Val Val Glu Val Asp Ala Ala Val Thr Pro Glu Glu Arg
            660                 665                 670

cac ctg tcc aag atg cag cag aac ggc tac gaa aat cca acc tac aag 2064
His Leu Ser Lys Met Gln Gln Asn Gly Tyr Glu Asn Pro Thr Tyr Lys
        675                 680                 685

ttc ttt gag cag atg cag aac tag 2088
Phe Phe Glu Gln Met Gln Asn
    690                 695

<210> 2

<211>  695
<212>  PRT
<213>  Homo sapiens

<400>  2

Met Leu Pro Gly Leu Ala Leu Leu Leu Leu Ala Ala Trp Thr Ala Arg
1               5                   10                  15

Ala Leu Glu Val Pro Thr Asp Gly Asn Ala Gly Leu Leu Ala Glu Pro
            20                  25                  30

Gln Ile Ala Met Phe Cys Gly Arg Leu Asn Met His Met Asn Val Gln
        35                  40                  45

Asn Gly Lys Trp Asp Ser Asp Pro Ser Gly Thr Lys Thr Cys Ile Asp
    50                  55                  60

Thr Lys Glu Gly Ile Leu Gln Tyr Cys Gln Glu Val Tyr Pro Glu Leu
65                  70                  75                  80

Gln Ile Thr Asn Val Val Glu Ala Asn Gln Pro Val Thr Ile Gln Asn
                85                  90                  95

Trp Cys Lys Arg Gly Arg Lys Gln Cys Lys Thr His Pro His Phe Val
            100                 105                 110

Ile Pro Tyr Arg Cys Leu Val Gly Glu Phe Val Ser Asp Ala Leu Leu
        115                 120                 125

Val Pro Asp Lys Cys Lys Phe Leu His Gln Glu Arg Met Asp Val Cys
    130                 135                 140

Glu Thr His Leu His Trp His Thr Val Ala Lys Glu Thr Cys Ser Glu
145                 150                 155                 160

Lys Ser Thr Asn Leu His Asp Tyr Gly Met Leu Leu Pro Cys Gly Ile
                165                 170                 175

Asp Lys Phe Arg Gly Val Glu Phe Val Cys Cys Pro Leu Ala Glu Glu
            180                 185                 190

Ser Asp Asn Val Asp Ser Ala Asp Ala Glu Glu Asp Asp Ser Asp Val
        195                 200                 205

Trp Trp Gly Gly Ala Asp Thr Asp Tyr Ala Asp Gly Ser Glu Asp Lys
    210                 215                 220

Val Val Glu Val Ala Glu Glu Glu Glu Val Ala Glu Val Glu Glu Glu
225                 230                 235                 240

Glu Ala Asp Asp Asp Glu Asp Asp Glu Asp Gly Asp Glu Val Glu Glu
                245                 250                 255

Glu Ala Glu Glu Pro Tyr Glu Glu Ala Thr Glu Arg Thr Thr Ser Ile
            260                 265                 270

Ala Thr Thr Thr Thr Thr Thr Thr Glu Ser Val Glu Glu Val Val Arg
        275                 280                 285

Val Pro Thr Thr Ala Ala Ser Thr Pro Asp Ala Val Asp Lys Tyr Leu
    290                 295                 300

Glu Thr Pro Gly Asp Glu Asn Glu His Ala His Phe Gln Lys Ala Lys
305                 310                 315                 320

Glu Arg Leu Glu Ala Lys His Arg Glu Arg Met Ser Gln Val Met Arg
                325                 330                 335

Glu Trp Glu Glu Ala Glu Arg Gln Ala Lys Asn Leu Pro Lys Ala Asp
            340                 345                 350

Lys Lys Ala Val Ile Gln His Phe Gln Glu Lys Val Glu Ser Leu Glu
        355                 360                 365

Gln Glu Ala Ala Asn Glu Arg Gln Gln Leu Val Glu Thr His Met Ala
    370                 375                 380

Arg Val Glu Ala Met Leu Asn Asp Arg Arg Arg Leu Ala Leu Glu Asn
385                 390                 395                 400

Tyr Ile Thr Ala Leu Gln Ala Val Pro Pro Arg Pro Arg His Val Phe
                405                 410                 415

Asn Met Leu Lys Lys Tyr Val Arg Ala Glu Gln Lys Asp Arg Gln His
            420                 425                 430

Thr Leu Lys His Phe Glu His Val Arg Met Val Asp Pro Lys Lys Ala
        435                 440                 445

Ala Gln Ile Arg Ser Gln Val Met Thr His Leu Arg Val Ile Tyr Glu
    450                 455                 460

Arg Met Asn Gln Ser Leu Ser Leu Leu Tyr Asn Val Pro Ala Val Ala
465                 470                 475                 480

Glu Glu Ile Gln Asp Glu Val Asp Glu Leu Leu Gln Lys Glu Gln Asn
                485                 490                 495

Tyr Ser Asp Asp Val Leu Ala Asn Met Ile Ser Glu Pro Arg Ile Ser
            500                 505                 510

Tyr Gly Asn Asp Ala Leu Met Pro Ser Leu Thr Glu Thr Lys Thr Thr
        515                 520                 525

Val Glu Leu Leu Pro Val Asn Gly Glu Phe Ser Leu Asp Asp Leu Gln
    530                 535                 540

Pro Trp His Ser Phe Gly Ala Asp Ser Val Pro Ala Asn Thr Glu Asn
545                 550                 555                 560

Glu Val Glu Pro Val Asp Ala Arg Pro Ala Ala Asp Arg Gly Leu Thr
                565                 570                 575

Thr Arg Pro Gly Ser Gly Leu Thr Asn Ile Lys Thr Glu Glu Ile Ser
            580                 585                 590

Glu Val Lys Met Asp Ala Glu Phe Arg His Asp Ser Gly Tyr Glu Val
        595                 600                 605

His His Gln Lys Leu Val Phe Phe Ala Glu Asp Val Gly Ser Asn Lys
    610                 615                 620

Gly Ala Ile Ile Gly Leu Met Val Gly Gly Val Val Ile Ala Thr Val
625                 630                 635                 640

Ile Val Ile Thr Leu Val Met Leu Lys Lys Lys Gln Tyr Thr Ser Ile
                645                 650                 655

His His Gly Val Val Glu Val Asp Ala Ala Val Thr Pro Glu Glu Arg
            660                 665                 670

His Leu Ser Lys Met Gln Gln Asn Gly Tyr Glu Asn Pro Thr Tyr Lys
        675                 680                 685

Phe Phe Glu Gln Met Gln Asn
    690                 695

<210>   3
<211>   3641
<212>   DNA
<213>   Homo sapiens

<220>
<221>   CDS
<222>   (195)..(2507)
<223>

<300>
<308>   GenBank/NM_000484
<309>   2004-01-30
<313>   (1)..(3641)

<400>   3
gctgactcgc ctggctctga gccccgccgc cgcgctcggg ctccgtcagt ttcctcggca      60
gcggtaggcg agagcacgcg gaggagcgtg cgcgggggcc ccgggagacg gcggcggtgg     120
cggcgcgggc agagcaagga cgcggcggat cccactcgca cagcagcgca ctcggtgccc     180
cgcgcagggt cgcg atg ctg ccc ggt ttg gca ctg ctc ctg ctg gcc gcc 230
tgg acg gct cgg gcg ctg gag gta ccc act gat ggt aat gct ggc ctg 278
ctg gct gaa ccc cag att gcc atg ttc tgt ggc aga ctg aac atg cac 326
atg aat gtc cag aat ggg aag tgg gat tca gat cca tca ggg acc aaa 374
acc tgc att gat acc aag gaa ggc atc ctg cag tat tgc caa gaa gtc 422
tac cct gaa ctg cag atc acc aat gtg gta gaa gcc aac caa cca gtg 470
acc atc cag aac tgg tgc aag cgg ggc cgc aag cag tgc aag acc cat 518
ccc cac ttt gtg att ccc tac cgc tgc tta gtt ggt gag ttt gta agt 566
gat gcc ctt ctc gtt cct gac aag tgc aaa ttc tta cac cag gag agg 614
atg gat gtt tgc gaa act cat ctt cac tgg cac acc gtc gcc aaa gag 662
aca tgc agt gag aag agt acc aac ttg cat gac tac ggc atg ttg ctg 710
ccc tgc gga att gac aag ttc cga ggg gta gag ttt gtg tgt tgc cca 758
ctg gct gaa gaa agt gac aat gtg gat tct gct gat gcg gag gag gat 806
gac tcg gat gtc tgg tgg ggc gga gca gac aca gac tat gca gat ggg 854
agt gaa gac aaa gta gta gaa gta gca gag gag gaa gaa gtg gct gag 902
gtg gaa gaa gaa gaa gcc gat gat gac gag gac gat gag gat ggt gat 950
gag gta gag gaa gag gct gag gaa ccc tac gaa gaa gcc aca gag aga 998
acc acc agc att gcc acc acc acc acc acc acc aca gag tct gtg gaa     1046
gag gtg gtt cga gag gtg tgc tct gaa caa gcc gag acg ggg ccg tgc     1094
cga gca atg atc tcc cgc tgg tac ttt gat gtg act gaa ggg aag tgt     1142
gcc cca ttc ttt tac ggc gga tgt ggc ggc aac cgg aac aac ttt gac     1190
aca gaa gag tac tgc atg gcc gtg tgt ggc agc gcc atg tcc caa agt     1238
tta ctc aag act acc cag gaa cct ctt gcc cga gat cct gtt aaa ctt     1286
cct aca aca gca gcc agt acc cct gat gcc gtt gac aag tat ctc gag     1334
aca cct ggg gat gag aat gaa cat gcc cat ttc cag aaa gcc aaa gag     1382
agg ctt gag gcc aag cac cga gag aga atg tcc cag gtc atg aga gaa     1430
tgg gaa gag gca gaa cgt caa gca aag aac ttg cct aaa gct gat aag     1478
aag gca gtt atc cag cat ttc cag gag aaa gtg gaa tct ttg gaa cag     1526
gaa gca gcc aac gag aga cag cag ctg gtg gag aca cac atg gcc aga     1574
gtg gaa gcc atg ctc aat gac cgc cgc cgc ctg gcc ctg gag aac tac     1622
atc acc gct ctg cag gct gtt cct cct cgg cct cgt cac gtg ttc aat     1670
atg cta aag aag tat gtc cgc gca gaa cag aag gac aga cag cac acc     1718
cta aag cat ttc gag cat gtg cgc atg gtg gat ccc aag aaa gcc gct     1766
cag atc cgg tcc cag gtt atg aca cac ctc cgt gtg att tat gag cgc     1814
atg aat cag tct ctc tcc ctg ctc tac aac gtg cct gca gtg gcc gag 1862
gag att cag gat gaa gtt gat gag ctg ctt cag aaa gag caa aac tat 1910
tca gat gac gtc ttg gcc aac atg att agt gaa cca agg atc agt tac 1958
gga aac gat gct ctc atg cca tct ttg acc gaa acg aaa acc acc gtg 2006
gag ctc ctt ccc gtg aat gga gag ttc agc ctg gac gat ctc cag ccg 2054
tgg cat tct ttt ggg gct gac tct gtg cca gcc aac aca gaa aac gaa 2102
gtt gag cct gtt gat gcc cgc cct gct gcc gac cga gga ctg acc act 2150
cga cca ggt tct ggg ttg aca aat atc aag acg gag gag atc tct gaa 2198
gtg aag atg gat gca gaa ttc cga cat gac tca gga tat gaa gtt cat 2246
cat caa aaa ttg gtg ttc ttt gca gaa gat gtg ggt tca aac aaa ggt 2294
gca atc att gga ctc atg gtg ggc ggt gtt gtc ata gcg aca gtg atc 2342
gtc atc acc ttg gtg atg ctg aag aag aaa cag tac aca tcc att cat 2390
cat ggt gtg gtg gag gtt gac gcc gct gtc acc cca gag gag cgc cac 2438
ctg tcc aag atg cag cag aac ggc tac gaa aat cca acc tac aag ttc 2486
ttt gag cag atg cag aac tag acccccgcca cagcagcctc tgaagttgga 2537
cagcaaaacc attgcttcac tacccatcgg tgtccattta tagaataatg tgggaagaaa 2597
caaacccgtt ttatgattta ctcattatcg ccttttgaca gctgtgctgt aacacaagta 2657
gatgcctgaa cttgaattaa tccacacatc agtaatgtat tctatctctc tttacatttt 2717
ggtctctata ctacattatt aatgggtttt gtgtactgta aagaatttag ctgtatcaaa 2777
ctagtgcatg aatagattct ctcctgatta tttatcacat agccccttag ccagttgtat 2837
attattcttg tggtttgtga cccaattaag tcctacttta catatgcttt aagaatcgat 2897
gggggatgct tcatgtgaac gtgggagttc agctgcttct cttgcctaag tattcctttc 2957
ctgatcacta tgcattttaa agttaaacat ttttaagtat ttcagatgct ttagagagat 3017
tttttttcca tgactgcatt ttactgtaca gattgctgct tctgctatat ttgtgatata 3077
ggaattaaga ggatacacac gtttgtttct tcgtgcctgt tttatgtgca cacattaggc 3137
attgagactt caagcttttc tttttttgtc cacgtatctt tgggtctttg ataaagaaaa 3197
gaatccctgt tcattgtaag cacttttacg gggcgggtgg ggaggggtgc tctgctggtc 3257
ttcaattacc aagaattctc caaaacaatt ttctgcagga tgattgtaca gaatcattgc 3317
ttatgacatg atcgctttct acactgtatt acataaataa attaaataaa ataaccccgg 3377
gcaagacttt tctttgaagg atgactacag acattaaata atcgaagtaa ttttgggtgg 3437
ggagaagagg cagattcaat ttttcttaac cagtctgaag tttcatttat gatacaaaag 3497
aagatgaaaa tggaagtggc aatataaggg gatgaggaag gcatgcctgg acaaaccctt 3557
cttttaagat gtgtcttcaa tttgtataaa atggtgtttt catgtaaata aatacattct 3617
tggaggagca aaaaaaaaaa aaaa 3641

<210> 4
<211> 11
<212> PRT
<213> Artificial

<220>
<223> Description of artificial sequence: Synthetic oligopeptide

<400> 4
Tyr Gly Arg Lys Lys Arg Arg Gln Arg Arg Arg
1               5                   10